# FASCICULE DE BREVET EUROPEEN

(11) **EP 3 356 325 B1**
(45) Date de publication et mention de la délivrance du brevet: **26.01.2022**
(21) Numéro de dépôt: 16785250.8
(22) Date de dépôt: 29.09.2016
(51) Int. Cl.: C07C 319/06, C07C 321/04, C12P 11/00, C12N 9/02

(54) **PROCÉDÉ DE PRODUCTION DE MERCAPTANS PAR HYDROGÉNOLYSE ENZYMATIQUE DE DISULFURES**
VERFAHREN ZUR HERSTELLUNG VON MERCAPTANEN DURCH DISULFIDENZYMHYDROGENOLYSE
METHOD FOR PRODUCING MERCAPTANS BY DISULFIDE ENZYME HYDROGENOLYSIS

(30) Priorité: 30.09.2015 FR 1559257
(43) Date de publication de la demande: 08.08.2018
(73) Titulaire: Arkema France, 92700 Colombes (FR)
(72) Inventeur: FREMY, Georges, 64390 Sauveterre De Bearn (FR); MASSELIN, Arnaud, 35400 Saint Malo (FR)
(74) Mandataire: Arkema Patent
(86) Numéro de dépôt international: PCT/FR2016/052479
(87) Numéro de publication internationale: WO 2017/055752

(56) Documents cités:
- US-A- 4 059 636
- US-A1- 2005 260 250
- SZAJEWSKI R P ET AL: "RATE CONSTANTS AND EQUILIBRIUM CONSTANTS FOR THIOL-DISULFIDE INTERCHANGE REACTIONS INVOLVING OXIDIZED GLUTATHIONE", JOURNAL OF THE AMERICAN CHEMICAL SOCIETY, AMERICAN CHEMICAL SOCIETY, US, vol. 102, no. 6, mars 1980 (1980-03), pages 2011-2025, XP002072983, ISSN: 0002-7863, DOI: 10.1021/JA00526A042
- MILLIS KEVIN K ET AL: "Oxidation/reduction potential of glutathione", THE JOURNAL OF ORGANIC CHEMISTRY, AMERICAN CHEMICAL SOCIETY, US, vol. 58, no. 15, janvier 1993 (1993-01), pages 4144-4146, XP009099411, ISSN: 0022-3263, DOI: 10.1021/JO00067A060
- MATTHEW J. G. STEWART ET AL: "Mycothiol disulfide reductase: solid phase synthesis and evaluation of alternative substrate analogues", ORGANIC & BIOMOLECULAR CHEMISTRY, vol. 6, no. 2, janvier 2008 (2008-01), page 385, XP055116173, ISSN: 1477-0520, DOI: 10.1039/b716380k
- DAVID A. KEIRE ET AL: "Kinetics and equilibria of thiol/disulfide interchange reactions of selected biological thiols and related molecules with oxidized glutathione", THE JOURNAL OF ORGANIC CHEMISTRY, vol. 57, no. 1, janvier 1992 (1992-01), pages 123-127, XP055257183, US ISSN: 0022-3263, DOI: 10.1021/jo00027a023
- MING DU ET AL: "Derivation of Oridonin with Bioreduction-Responsive Disulfide Bond", CHINESE JOURNAL OF CHEMISTRY, vol. 32, no. 5, 22 mai 2014 (2014-05-22), pages 448-453, XP055257871, CN ISSN: 1001-604X, DOI: 10.1002/cjoc.201300761
- SHUANG LIU ET AL: "Oligomeric Hydrogels Self-Assembled from Reduction-Controlled Condensation", ANGEWANDTE CHEMIE INTERNATIONAL EDITION, vol. 54, no. 12, 16 mars 2015 (2015-03-16) , pages 3639-3642, XP055257879, DE ISSN: 1433-7851, DOI: 10.1002/anie.201409952

## Description

La présente invention concerne un procédé de production par catalyse enzymatique de mercaptans, en particulier de méthylmercaptan, à partir de disulfures, en particulier de disulfure de diméthyle, et à l'aide de composés organiques réducteurs.

Les mercaptans sont d'une grande utilité dans de très nombreux domaines, par exemple comme arômes, odorisants pour gaz, agents de transfert de chaîne en polymérisation, matières premières pour l'industrie pharmaceutique ou cosmétique, pour la synthèse d'antioxydants, d'additifs extrême pression ou anti-usure pour la lubrification. Ces exemples ne sont en aucun cas limitatifs des utilisations des mercaptans connus aujourd'hui et qui peuvent être préparés grâce au procédé de l'invention.

En particulier, le premier des mercaptans, le méthylmercaptan (CH₃SH), présente un grand intérêt industriel, en particulier comme matière première de la synthèse de méthionine, acide aminé essentiel très largement utilisé dans l'alimentation animale. Le méthylmercaptan est également une matière première très largement utilisée pour la synthèse de nombreuses autres molécules.

Les mercaptans peuvent être synthétisés par de nombreuses méthodes telles que la sulfhydratation d'alcools, l'addition catalytique ou photochimique d'hydrogène sulfuré sur des composés organiques insaturés, la substitution à l'aide d'hydrogène sulfuré d'halogénures, d'époxydes ou de carbonates organiques, et autres.

En particulier, le méthylmercaptan est couramment produit en gros tonnage industriellement à partir de méthanol et de sulfure d'hydrogène selon la réaction (1) :

CH₃OH + H₂S → CH₃SH + H₂O (1)

Ces procédés présentent les inconvénients de nécessiter du méthanol (CH₃OH), de synthétiser l'hydrogène sulfuré (H₂S, à partir d'hydrogène et de soufre par exemple, d'où la nécessité de synthétiser de l'hydrogène également) et conduisent à des sous-produits de type éther diméthylique (CH₃OCH₃), sulfure de diméthyle (CH₃SCH₃) et produits de craquages et de l'eau, ce qui sous-entend de nombreuses étapes de purification du méthylmercaptan.

On trouvera la description de procédés basés sur ces réactions, à titre d'exemples, dans les demandes de brevets telles que WO2013092129, WO2008118925, WO2007028708, WO2006015668, WO2004096760.

Il peut se révéler intéressant économiquement (pour éviter la synthèse du méthanol) de vouloir produire le méthylmercaptan à partir de monoxyde de carbone, d'hydrogène et de sulfure d'hydrogène selon le schéma de synthèse (2) suivant :

CO + 2 H₂ + H₂S → CH₃SH + H₂O (2)

Cependant, ces procédés présentent les inconvénients de nécessiter du gaz de synthèse (CO/H₂) et donc de procéder à un reformage à la vapeur d'une source hydrocarbonée, d'avoir les bonnes proportions entre CO et H₂ donc de pouvoir ajuster le ratio CO/H₂ avec la réaction dite du « gaz à l'eau » (CO + H₂O → CO₂ + H₂), et de synthétiser l'H₂S.

Ces procédés conduisent également généralement à de fortes proportions de CO₂ en tant que sous-produit, ainsi qu'à du méthane, du sulfure de diméthyle et de l'eau. On trouvera des descriptions de ces procédés à titre d'exemple dans les demandes de brevets US2010286448, US2010094059, US2008293974, US2007213564.

D'autres procédés encore ont été décrits et combinent différentes réactions telles que :
- Formation de CS₂ et d'H₂S à partir de méthane et de soufre (3) :

   CH₄ + 4 S → CS₂ + 2 H₂S (3)
- Hydrogénation du CS₂ (4) :

   CS₂ + 3 H₂ → CH₃SH + H₂S (4)

Il est également possible d'utiliser l'H₂S excédentaire des réactions (3) et (4) dans les réactions avec du méthanol (réaction 1) ou du gaz de synthèse (réaction 2) pour donner à nouveau du méthylmercaptan.

Ces procédés combinent évidemment les inconvénients décrits pour les réactions (1) et (2) avec la difficulté supplémentaire d'avoir de l'hydrogène excédentaire pour effectuer la réaction (4). On trouvera des descriptions de ces procédés dans les demandes de brevets US2011015443 ou, plus spécifiquement sur la réaction (4), dans la demande WO2010046607.

La demande WO200196290 propose un procédé de synthèse de méthylmercaptan directement à partir de méthane et d'H₂S avec coproduction d'hydrogène. Cette réaction directe entre le méthane et l'H₂S se fait à l'aide d'un plasma pulsé avec décharge en couronne. Cette demande ne décrivant aucun exemple de synthèse, il peut paraître difficile d'imaginer un procédé de synthèse industriel de méthylmercaptan à grande échelle avec cette technologie. De plus ce procédé nécessite la synthèse de l'H₂S si celui-ci n'est pas disponible.

La demande de brevet EP0649837 propose quant à elle un procédé de synthèse de méthylmercaptan par hydrogénolyse catalytique, avec des sulfures de métaux de transition, du disulfure de diméthyle avec de l'hydrogène. Ce procédé, bien que performant, nécessite des températures relativement élevées de l'ordre de 200°C pour obtenir des productivités intéressantes industriellement.

L'homme de l'art sait également qu'il est possible de préparer du méthylmercaptan par acidification d'une solution aqueuse de méthylmercaptide de sodium (CH₃SNa). Cette méthode présente l'inconvénient majeur de produire de grandes quantités de sels, tels que le chlorure de sodium ou le sulfate de sodium, selon que l'on utilise l'acide chlorhydrique ou l'acide sulfurique. Ces solutions aqueuses salines sont souvent très difficiles à traiter et les traces de produits malodorants qui subsistent font que cette méthode est difficilement envisageable sur le plan industriel.

Les procédés de synthèse des mercaptans supérieurs au méthylmercaptan présentent également de nombreux inconvénients. Ainsi la substitution des alcools par l'hydrogène sulfuré nécessite des températures et souvent des pressions élevées, et conduit à des sous-produits non-désirés de type oléfines, éthers et sulfures.

L'addition catalytique ou photochimique de l'hydrogène sulfuré sur des composés insaturés se fait généralement dans des conditions légèrement plus douces que précédemment mais conduit également à de nombreux sous-produits formés par isomérisation de la matière première, par addition non-régiosélective ou par double addition qui donne des sulfures. Enfin, la substitution de dérivés halogénés conduit à des procédés qui génèrent beaucoup d'effluents et de rejets salins difficilement compatibles avec des procédés industriels.

Dans le domaine de la biologie, plusieurs articles décrivent l'utilisation du couple glutathion/disulfure de glutathion (ou système GSH/GSSG) dans des réactions d'oxydo-réductions. L'article de R. P. Szajewski, (1980, Thiol-Disulfide Interchange reactions, American Chemical Society, p.2011-2025) décrit des thiols utilisés en tant que réducteurs du glutathion et transformés en disulfures et K. K. Millis et al., (J. Org. Chem. 1993, 58, 4144-4146) traite du potentiel de demi-cellule du système rédox glutathion/disulfure de glutathion. Un mélange comprenant du NADPH/NADP+, du GSH/GSSG et de la glutathion réductase est réalisé. Ming Du et al., (Chin. J. Chem. 2014, 32, 448-453) décrit l'utilisation de glutathion pour former un analogue de l'oridonine en réduisant un pont disulfure, à l'intérieur de cellules tumorales.

Shuang Liu et al. (Angew. Chem. Int. Ed. 2015, 54, 3639-3642) décrit la formation d'hydrogels oligomériques qui s'auto-assemblent par réduction de ponts disulfures grâce au glutathion. US 2005/0260250 traite de compléments alimentaires, permettant d'obtenir des thiols biodisponibles une fois ingérés grâce à plusieurs cycles anti-oxydants transmembranaires comprenant le glutathion.

Enfin, M. J. G. Stewart (Org. Biomol. Chem., 2008, 6, 385-390) décrit la synthèse et l'évaluation d'analogues du mycothiol, qui sont oxydés sous leur forme disulfure pour être testés en tant que substrat de l'enzyme mycothiol disulfure réductase.

La présente invention a pour objet de proposer un nouveau procédé de préparation de mercaptans, en particulier de méthylmercaptan, qui ne présente pas les inconvénients décrits dans les procédés connus de l'art antérieur et précédemment détaillés.

Plus particulièrement, la présente invention a comme premier objet le procédé de préparation d'un mercaptan de formule R-SH, comprenant au moins les étapes de :
a) préparation d'un mélange comprenant :
   1) un disulfure de formule R-S-S-R',
   2) une quantité catalytique d'acide aminé porteur d'un groupement thiol ou d'un peptide à groupement thiol,
   3) une quantité catalytique d'une enzyme catalysant la réduction du pont disulfure créé entre deux équivalents dudit acide aminé porteur d'un groupement thiol ou dudit peptide à groupement thiol,
   4) une quantité catalytique d'une enzyme catalysant la déshydrogénation du composé organique réducteur impliqué dans l'étape b),
   5) une quantité catalytique d'un cofacteur commun aux deux enzymes catalysant la réduction et la déshydrogénation,
b) ajout d'un composé organique réducteur en quantité stœchiométrique par rapport au disulfure de formule R-S-S-R',
c) conduite de la réaction enzymatique,
d) récupération du mercaptan de formule R-SH et du mercaptan de formule R'-SH,
e) séparation et purification éventuelle du mercaptan de formule R-SH et du mercaptan de formule R'-SH,
   dans lequel R et R', identiques ou différents, représentent indépendamment l'un de l'autre, un radical hydrocarboné, linéaire ou ramifié, comportant de 1 à 20 atomes de carbone, ladite chaîne étant saturée ou porteuse d'une ou plusieurs insaturations sous forme de double(s) ou triple(s) liaison(s), ou
   R et R' forment ensemble et avec les atomes de soufre qui les portent une molécule cyclique comportant de 4 à 22 atomes.

D'une manière générale, l'enzyme catalysant la réduction du pont disulfure créé entre deux équivalents dudit acide aminé porteur d'un groupement thiol ou dudit peptide à groupement thiol est une enzyme réductase. Le terme « réductase » est utilisé dans la suite de la description pour l'explication de la présente invention. De manière similaire, l'enzyme catalysant la déshydrogénation du composé organique réducteur impliqué dans l'étape b) est généralement dénommée enzyme déshydrogénase, le terme « déshydrogénase » étant choisi dans la suite de la description pour l'explication de la présente invention.

Parmi les cofacteurs communs aux deux enzymes catalysant la réduction et la déshydrogénation (la réductase et la déshydrogénase), on peut citer à titre d'exemples non limitatifs les cofacteurs flaviniques, et les cofacteurs nicotiniques. On préfère utiliser les cofacteurs nicotiniques et plus particulièrement la Nicotinamide Adénine Dinucléotide (NAD), ou mieux encore la Nicotinamide Adénine Dinucléotide Phosphate (NADPH). Les cofacteurs listés ci-dessus sont avantageusement utilisés sous leurs formes réduites (par exemple NADPH, H+) et/ou leurs formes oxydées (par exemple NADP+), c'est-à-dire qu'ils peuvent être ajoutés sous ces formes réduites et/ou oxydées dans le milieu réactionnel.

Dans un mode de réalisation de l'invention, l'acide aminé porteur d'un groupement thiol et/ou le peptide porteur d'un groupement thiol peut être sous la forme du disulfure dudit acide aminé et/ou dudit peptide respectivement, par exemple glutathion sous forme de disulfure de glutathion.

L'organisation et l'ordre des ajouts des différents composants des étapes a) et b) du précédé défini ci-dessus peuvent être réalisés de différentes manières. Dans tous les cas, la réaction enzymatique de l'étape c) est déclenchée par ajout d'un des composants du système catalytique : soit une enzyme, soit un des composés ajoutés en quantité stœchiométrique (disulfure ou composé organique réducteur) soit un des composés ajoutés en quantité catalytique (acide aminé porteur d'un groupement thiol ou peptide à groupement thiol ou du disulfure correspondant auxdites molécules ou bien encore le cofacteur).

Plus particulièrement encore, la présente invention a comme objet le procédé de préparation d'un mercaptan de formule R-SH, comprenant au moins les étapes de :
a') préparation d'un mélange comprenant :
   - un disulfure de formule R-S-S-R',
   - une quantité catalytique d'acide aminé porteur d'un groupement thiol ou d'un peptide à groupement thiol,
   - une quantité catalytique d'enzyme réductase correspondant audit acide aminé porteur d'un groupement thiol ou audit peptide à groupement thiol,
   - une quantité catalytique de NADPH,
b') ajout d'un composé organique réducteur en quantité stœchiométrique par rapport au disulfure et DMDS) avec une quantité catalytique de l'enzyme déshydrogénase correspondante,
c') conduite de la réaction enzymatique,
d') récupération du mercaptan de formule R-SH et du mercaptan de formule R'-SH,
e') séparation et purification éventuelle du mercaptan de formule R-SH et du mercaptan de formule R'-SH

Dans la formule générale R-S-S-R', R et R', identiques ou différents, représentent indépendamment l'un de l'autre, un radical hydrocarboné, linéaire ou ramifié, comportant de 1 à 20 atomes de carbone, ladite chaîne étant saturée ou porteuse d'une ou plusieurs insaturations sous forme de double(s) ou triple(s) liaison(s). R et R' peuvent également former ensemble et avec les atomes de soufre qui les portent une molécule cyclique comportant de 4 à 22 atomes, de préférence de 5 à 10 atomes.

Selon un aspect préféré, les radicaux R et R', identiques ou différents, sont choisis indépendamment l'un de l'autre, parmi les radicaux alkyle, alkylaryle, comportant de 1 à 20 atomes de carbone, de préférence de 1 à 12 atomes de carbone de préférence encore de 1 à 6 atomes de carbone, linéaires ou ramifiés, saturés ou non, et éventuellement fonctionnalisés par une ou plusieurs fonctions choisies, de manière non limitative et à titre d'exemples, parmi les fonction alcool, aldéhyde, cétone, acide, amide, nitrile, ester ou encore les fonctions porteuses de soufre, phosphore, silicium ou halogène.

Le disulfure de formule R-S-S-R' est susceptible d'être réduit, selon le procédé de l'invention, en mercaptan de formule R-SH et en mercaptan de formule R'-SH. Lorsque R est différent de R', on parle de disulfure dissymétrique, et lorsque R et R' sont identiques, on parle de disulfure symétrique. Dans le cas de disulfures symétriques R-S-S-R, le procédé de l'invention conduit à un mercaptan de formule R-SH. Selon un aspect tout particulièrement préféré de l'invention, on met en œuvre du disulfure de diméthyle (DMDS) dans le but de produire du méthylmercaptan CH₃SH.

Dans le cas de disulfures dissymétriques R-S-S-R', le procédé de l'invention conduit à un mélange des mercaptans de formules R-SH et R'-SH qui peut être soit utilisé tel quel ou bien soumis à une ou plusieurs opérations de séparation bien connues de l'homme du métier, par exemple la distillation.

Il est également possible de mettre en œuvre dans le procédé de l'invention des mélanges de un ou plusieurs disulfures, symétriques et/ou dissymétriques. Des mélanges de disulfures possibles peuvent comprendre les DSO (« DiSulfide Oils » en langue anglaise), lesdits DSO trouvant ainsi une possibilité de valorisation tout à fait intéressante.

Selon le procédé de l'invention, le ou les mercaptans produits sont généralement récupérés sous la forme d'un solide, d'un liquide et/ou d'un gaz.

Le procédé de production selon l'invention est basé sur la réduction enzymatique des disulfures, en particulier du disulfure de diméthyle, avec un composé organique réducteur qui est un donneur d'hydrogène, ainsi qu'il sera défini plus loin, selon la réaction suivante, illustrée avec le disulfure de diméthyle conduisant au méthylmercaptan, en utilisant du glucose comme composé organique réducteur (donneur d'hydrogène) :

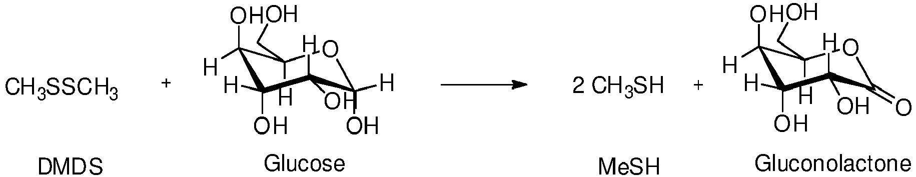

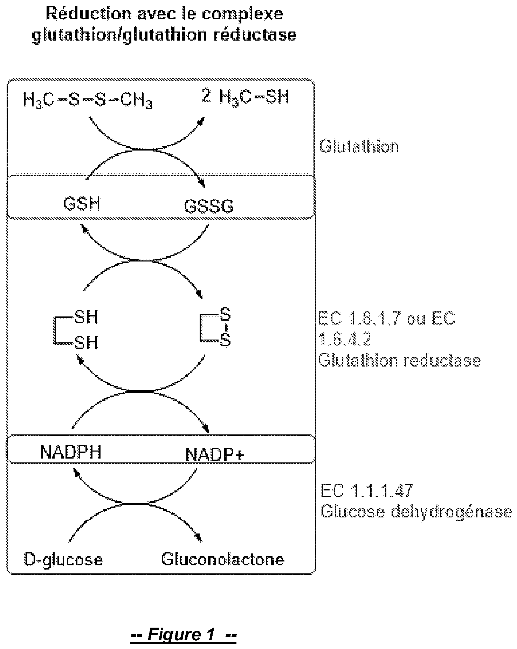

Il a maintenant été découvert que cette réaction est facilement catalysée par le système enzymatique mettant en œuvre un acide aminé à groupement thiol ou un peptide à groupement thiol, par exemple le glutathion, sous forme de complexe (acide aminé ou peptide)/enzyme réductase correspondante, régénéré par le composé organique donneur d'hydrogène, comme décrit à la Figure 1 annexée.

Ainsi selon l'illustration de la Figure 1, le peptide (exemple représenté le glutathion) réduit le disulfure (DMDS représenté) en mercaptan (méthylmercaptan représenté) en se transformant en peptide à pont disulfure (disulfure de glutathion représenté). L'enzyme réductase (« glutathion réductase » représentée, numéros de classification enzymatique EC 1.8.1.7 ou EC 1.6.4.2) régénère le peptide (glutathion) et cette même enzyme est régénérée par un complexe enzymatique oxydo-réducteur bien connu de l'homme du métier, par exemple le complexe NADPH/NADP+ (Nicotine adénine dinucléotide phosphate (forme réduite et forme oxydée)). À son tour NADP+ est régénéré en NADPH par l'intermédiaire de l'enzyme déshydrogénase correspondante au composé organique réducteur employé (ici la «glucose déshydrogénase» EC 1.1.1.47) grâce audit composé organique réducteur (glucose représenté) qui fournit de l'hydrogène (donneur d'hydrogène) en se transformant en sa forme oxydée (ici la gluconolactone).

En d'autres termes, l'enzyme catalysant la réduction (« glutathion réductase » représentée, avec les exemples de numéros de classification enzymatique EC 1.8.1.7 ou EC 1.6.4.2) régénère le peptide (glutathion) tout en oxydant le cofacteur (« NADPH,H+ » représenté). La forme oxydée (« NADP+ » représenté) est alors réduite à l'aide d'un complexe enzymatique oxydo-réducteur, dit « de recyclage », bien connu de l'homme de métier et comprenant l'enzyme déshydrogénase impliquée (« glucose déshydrogénase » représentée, avec l'exemple de numéro de classification enzymatique EC 1.1.1.47) et la molécule organique réductrice (« glucose » représenté). La forme oxydée du composé organique réducteur est alors obtenue (« gluconolactone » représentée).

Selon un mode de réalisation tout particulièrement adapté, le système glutathion/disulfure de glutathion associé à l'enzyme glutathion réductase permet selon la présente invention de réduire le DMDS en méthylmercaptan.

Le glutathion est un tripeptide largement utilisé en biologie. Cette espèce sous forme réduite (glutathion) ou oxydée (disulfure de glutathion) forme un couple d'oxydo-réduction important dans les cellules. Ainsi le glutathion est vital pour supprimer les métaux lourds des organismes. Ainsi par exemple la demande WO05107723 décrit une formulation où le glutathion est utilisé pour former une préparation chélatante, le brevet US4657856 enseigne que le glutathion permet également de détruire les peroxydes comme l'H₂O₂ en H₂O via la glutathion peroxydase. Enfin le glutathion permet également de réduire les ponts disulfures présents dans les protéines (Rona Chandrawati, « Triggered Cargo Release by Encapsulated Enzymatic Catalysis in Capsosomes », Nano Lett., (2011), vol. 11, 4958-4963).

Selon le procédé de l'invention, une quantité catalytique d'acide aminé porteur d'un groupement thiol ou d'un peptide à groupement thiol, est mise en œuvre pour la production de mercaptans à partir de disulfures.

Parmi les acides aminés porteurs de groupement thiol utilisables dans le procédé de la présente invention, on peut citer, à titre d'exemples non limitatifs la cystéine et l'homocystéine. Les systèmes enzymatiques oxydo-réducteurs utilisés pouvant régénérer le cycle catalytique de la même façon, sont dans ces cas le système cystéine/cystine-réductase EC 1.8.1.6, et homocystéine/homocystéineréductase.

Parmi les peptides porteurs de groupement thiol utilisables dans le procédé de la présente invention, on peut citer, à titre d'exemples non limitatifs le glutathion et la thiorédoxine. Le système glutathion/glutathion réductase, décrit précédemment, peut ainsi être remplacé par le système thiorédoxine (CAS no. 52500-60-4)/thioredoxine réductase (EC 1.8.1.9 ou EC 1.6.4.5).

Le glutathion et le système glutathion/glutathion réductase sont tout particulièrement préférés pour la présente invention, en raison de la facilité d'approvisionnement et des coûts de ces composés.

Parmi les composés organiques réducteurs qui peuvent être utilisés dans le cadre de la présente invention, les composés donneurs d'hydrogène sont tout particulièrement préférés, et parmi ceux-ci, les composés tout à fait adaptés sont les composés organiques réducteurs donneurs d'hydrogène porteurs de fonction hydroxyle, tels que les alcools, les polyols, les sucres et autres.

L'enzyme utilisée est une enzyme apte à déshydrogéner le composé porteur d'hydrogène, par exemple une alcool-déshydrogénase. Le glucose est un sucre tout particulièrement bien adapté pour être mis en œuvre dans le procédé de la présente invention, avec la glucose-déshydrogénase, pour donner la gluconolactone.

Dans le procédé selon l'invention seuls les disulfure(s) et le glucose sont utilisés en quantité stœchiométrique, tous les autres composants (acide aminé ou peptide, cofacteur (par exemple NADPH) et les 2 enzymes) sont utilisés en quantité catalytique.

Les avantages que procure le procédé de l'invention sont nombreux. Parmi ces avantages, on peut citer la possibilité de travailler en solution aqueuse ou hydroorganique, dans des conditions très douces de température et de pression et dans des conditions de pH proches de la neutralité. Toutes ces conditions sont typiques d'un procédé biocatalytique dit « vert » ou « durable ».

Un autre avantage lorsque le procédé met en œuvre du disulfure de diméthyle est que le méthylmercaptan produit, qui est à l'état gazeux dans les conditions de réaction, sort du milieu réactionnel au fur et à mesure de sa formation. Le méthylmercaptan peut donc être directement utilisé à la sortie du réacteur dans une application plus en aval. Il peut aussi être facilement liquéfié par cryogénie par exemple si on souhaite l'isoler. On peut éventuellement accélérer son départ du milieu réactionnel en introduisant par bullage un léger débit d'azote.

Le disulfure de diméthyle (DMDS) peut être produit sur un autre site à partir de méthylmercaptan et d'un oxydant tels que l'oxygène, le soufre ou l'eau oxygénée par exemple, ou encore à partir de sulfate de diméthyle et de disulfure de sodium. Le DMDS peut aussi provenir d'une source de « DiSulfide Oils » (DSO), comme indiqué précédemment, puis purifié par exemple par distillation réactive, comme décrit dans la demande WO2014033399. À noter que les DSO peuvent aussi être utilisés tels quels, sans nécessité de purification entre les différents disulfures les composant. On obtient alors un mélange de mercaptans en appliquant le procédé de l'invention.

Lorsque le DMDS est utilisé comme disulfure, le procédé selon l'invention est peut alors être considéré comme un procédé permettant d'éviter le transport de méthylmercaptan de son site de production par les voies existantes industrielles, vers son site d'utilisation, s'ils sont différents. En effet, le méthylmercaptan est un gaz à température ambiante, toxique et fortement malodorant ce qui complique énormément son transport déjà très réglementé contrairement au DMDS. Le procédé décrit dans la présente invention peut donc être utilisé pour produire du méthylmercaptan directement sur le site d'utilisation de ce dernier.

Le DMDS étant consommé dans la réaction et le méthylmercaptan sortant du milieu réactionnel au fur et à mesure de sa formation, seul le produit de déshydrogénation du composé organique réducteur, par exemple la gluconolactone, s'accumule dans le milieu réactionnel, dans l'hypothèse d'une alimentation en continu de glucose et de DMDS. Lorsque la concentration en gluconolactone dépasse la saturation dans les conditions de réaction, cette dernière va précipiter et pourra alors être isolée du milieu réactionnel par tout moyen connu de l'homme du métier.

La gluconolactone peut avoir plusieurs utilisations. Elle est par exemple utilisée en tant qu'additif alimentaire connu sous le sigle E575. La gluconolactone s'hydrolyse en milieux aqueux acides pour former de l'acide gluconique également utilisé comme additif alimentaire (E574). La gluconolactone est également utilisée pour la production de tofu (cf. CN103053703) pour l'industrie alimentaire.

La gluconolactone peut notamment et avantageusement, dans le sens où elle représente le « déchet » du procédé selon la présente invention, remplacer le glucose dans une éventuelle réaction de fermentation pour produire soit du bioéthanol soit toute autre molécule issue de fermentation de sucre ou d'amidon.

Certaines bactéries peuvent en effet utiliser en fermentation la gluconolactone comme source de carbone, comme décrit par J. P. van Dijken, « Novel pathway for alcoholic fermentation of gluconolactone in the yeast Saccharomyces bulderi », J. Bacteriol., (2002), Vol. 184(3), 672-678.

D'autres sucres encore peuvent être utilisés dans le procédé de l'invention, et par exemple il est possible de remplacer le système glucose/gluconolactone/ glucose-déshydrogénase par le système suivant : glucose-6-phosphate/ 6-phosphoglucono-δ-lactone/Glucose-6-phosphate déshydrogénase (EC 1.1.1.49).

Il est également possible dans le procédé de l'invention d'utiliser un alcool en remplacement du sucre, et ainsi utiliser à la place du système glucose/gluconolactone/glucose déshydrogénase, le système général suivant : Alcool/cétone ou aldéhyde/ alcool-déshydrogénase (EC 1.1.1) et plus particulièrement le système isopropanol/acétone/isopropanol-déshydrogénase (EC 1.1.1.80).

En effet, ce système permet d'obtenir, quand on utilise du DMDS comme disulfure, un mélange constitué de méthylmercaptan (MeSH) et d'acétone qui sort du milieu réactionnel (donc pas d'accumulation d'aucun produit). Le MeSH et l'acétone peuvent être facilement séparés par une simple distillation si on le désire. Dans le cas d'autres disulfures, en fonction du point d'ébullition du mercaptan formé et de sa solubilité dans le milieu réactionnel, l'acétone peut facilement être retirée du milieu et le mercaptan peut éventuellement décanter du milieu réactionnel pour être aisément séparé.

De manière générale, la température de la réaction est comprise dans un domaine allant de 10°C à 50°C, de préférence entre 15°C et 45°C, de préférence encore entre 20°C et 40°C.

Le pH de la réaction peut être compris entre 6 et 8, de préférence entre 6,5 et 7,5. Le pH du milieu réactionnel peut être ajusté au moyen d'un tampon. De façon tout à fait préférée, on choisira le pH du tampon phosphate à 7,3.

La pression utilisée pour la réaction peut aller d'une pression réduite par rapport à la pression atmosphérique à plusieurs bars (plusieurs centaines de kPa), selon les réactifs utilisés et le matériel utilisé. Dans le cas où le DMDS est utilisé comme disulfure, une pression réduite peut en effet permettre un dégazage plus rapide du méthylmercaptan formé mais présente l'inconvénient d'augmenter les pressions de vapeur saturante de l'eau et du DMDS, polluant un peu plus le méthylmercaptan formé. De façon préférée, on utilisera une pression allant de la pression atmosphérique à 20 bars (2 MPa) et de façon encore plus préférée on travaillera sous une pression allant de la pression atmosphérique à 3 bars (300 kPa).

Le procédé selon l'invention peut être réalisé en batch ou en continu, dans un réacteur en verre ou en métal suivant les conditions opératoires retenues et les réactifs utilisés.

Le ratio molaire composé organique réducteur/disulfure idéal est la stœchiométrie (ratio molaire = 1) mais peut varier de 0,01 à 100 si l'homme du métier y trouve un intérêt quelconque telle qu'une addition en continu du disulfure alors que le composé réducteur est introduit dès le départ dans le réacteur. De façon préférée ce ratio molaire est choisi entre 0,5 et 5 globalement sur l'ensemble de la réaction.

Les éléments présents en quantité catalytique dans le mélange préparé à l'étape a) ci-dessus (acide aminé porteur d'un groupement thiol ou d'un peptide à groupement thiol, enzyme réductase, cofacteur tel que par exemple NADPH) sont aisément accessibles dans le commerce ou peuvent être préparés selon des techniques bien connues de l'homme du métier. Ces différents éléments peuvent se présenter sous forme solide ou liquide et peuvent très avantageusement être mis en solution dans l'eau pour être mis en œuvre dans le procédé de l'invention. Les enzymes utilisées peuvent également être greffées sur support (cas des enzymes supportées).

La solution aqueuse de complexe enzymatique comprenant l'acide aminé ou le peptide peut également être reconstituée par les méthodes connues par l'homme de métier, par exemple par perméabilisation de cellules qui contiendraient ces éléments. Cette solution aqueuse dont une composition est donnée dans l'exemple 1 suivant peut être utilisée dans des teneurs en masse comprises entre 0,01% et 20% par rapport au poids total du milieu réactionnel. De façon préférée on utilisera une teneur comprise entre 0,5% et 10%.

Il est également décrit, mais ne faisant pas partie de l'invention, l'utilisation d'une solution aqueuse de complexe enzymatique comprenant un acide aminé porteur d'une fonction thiol tel que défini précédemment ou d'un peptide porteur d'une fonction thiol tel que défini précédemment, pour la synthèse d'un mercaptan à partir d'un disulfure.

Le mélange pouvant être utilisé pour l'étape a) du procédé décrit précédemment et comprenant :
1) du diméthyldisulfure,
2) une quantité catalytique d'acide aminé porteur d'un groupement thiol ou d'un peptide à groupement thiol,
3) une quantité catalytique d'une enzyme catalysant la réduction du pont disulfure créé entre deux équivalents dudit acide aminé porteur d'un groupement thiol ou audit peptide à groupement thiol,
4) une quantité catalytique d'une enzyme catalysant la déshydrogénation d'un composé organique réducteur,
5) une quantité catalytique d'un cofacteur commun aux deux enzymes catalysant la réduction et la déshydrogénation,
est nouveau et à ce titre fait partie de la présente invention.

Dans un mode de réalisation de l'invention, l'acide aminé porteur d'un groupement thiol et/ou le peptide porteur d'un groupement thiol peut être sous la forme du disulfure dudit acide aminé et/ou dudit peptide respectivement.

Plus particulièrement, ledit mélange comprend :
- du diméthyldisulfure,
- une quantité catalytique d'acide aminé porteur d'un groupement thiol ou d'un peptide à groupement thiol,
- une quantité catalytique d'enzyme réductase correspondant audit acide aminé porteur d'un groupement thiol ou audit peptide à groupement thiol, et
- une quantité catalytique de NADPH.

On comprendra mieux l'invention avec les exemples suivants non limitatifs par rapport à la portée de l'invention.

### EXEMPLE 1 :

Dans un réacteur contenant 150 mL de tampon phosphate à 0,1 mol/L à pH 7,30, sont introduits 10 mL de complexe enzymatique au glutathion et 19,2 g (0,1 mol) de glucose. La solution de complexe enzymatique contient : 185 mg (0,6 mmol) de glutathion, 200 U de glutathion-réductase, 50 mg (0,06 mmol) de NADPH et 200 U de glucose-déshydrogénase. Le milieu réactionnel est porté à 25°C sous agitation mécanique. Un premier prélèvement est effectué t = 0. Par la suite, le disulfure de diméthyle (9,4 g, 0,1 mol) est placé dans une burette et ajouté au goutte à goutte dans le réacteur, la réaction commence. Un courant d'azote est placé dans le réacteur. Une analyse en chromatographie en phase gazeuse des gaz sortant du réacteur montre quasi essentiellement la présence d'azote et de méthylmercaptan (quelques traces d'eau). Ces gaz de sortie sont piégés dans de l'hydroxyde de sodium (soude) à 20% dans l'eau. Le DMDS est introduit en 6 heures et la réaction est suivie par dosage potentiométrique en argentimétrie du sel de sodium du méthylmercaptan dans le piège en sortie de réacteur. L'analyse finale montre que le DMDS a été converti de façon quantitative en méthylmercaptan. De plus, une analyse finale en chromatographie en phase gazeuse du milieu réactionnel confirme l'absence de DMDS, et par UPLC/masse on trouve des traces de glucose et la présence quasi exclusive de gluconolactone.

### EXEMPLE 2 :

Au milieu réactionnel de l'exemple 1, on réintroduit 19,2 g (0,1 mol) de glucose en une seule fois et 9,4 g (0,1 mol) de DMDS au goutte à goutte en 6 heures. Le suivi de la réaction se fait de la même façon que dans l'exemple 1 après avoir changé la solution de soude à 20% en sortie du réacteur. Les analyses en fin de réaction confirment la disparition complète du DMDS totalement converti en méthylmercaptan retrouvé sous forme de sel de sodium dans la solution de soude. Seule la gluconolactone est analysée et trouvée dans le milieu réactionnel en fin de réaction. Cet exemple montre la robustesse du système catalytique par sa reproductibilité.

## Revendications

1. Procédé de préparation d'un mercaptan de formule R-SH, comprenant au moins les étapes de :
a) préparation d'un mélange comprenant :
1) un disulfure de formule R-S-S-R',
2) une quantité catalytique d'acide aminé porteur d'un groupement thiol ou d'un peptide à groupement thiol,
3) une quantité catalytique d'une enzyme catalysant la réduction du pont disulfure créé entre deux équivalents dudit acide aminé porteur d'un groupement thiol ou dudit peptide à groupement thiol,
4) une quantité catalytique d'une enzyme catalysant la déshydrogénation du composé organique réducteur impliqué dans l'étape b),
5) une quantité catalytique d'un cofacteur commun aux deux enzymes catalysant la réduction et la déshydrogénation,
b) ajout d'un composé organique réducteur en quantité stœchiométrique par rapport au disulfure de formule R-S-S-R',
c) conduite de la réaction enzymatique,
d) récupération du mercaptan de formule R-SH et du mercaptan de formule R'-SH,
e) séparation éventuelle et purification éventuelle du mercaptan de formule R-SH et/ou du mercaptan de formule R'-SH ;
dans lequel R et R', identiques ou différents, représentent indépendamment l'un de l'autre, un radical hydrocarboné, linéaire ou ramifié, comportant de 1 à 20 atomes de carbone, ladite chaîne étant saturée ou porteuse d'une ou plusieurs insaturations sous forme de double(s) ou triple(s) liaison(s), ou
R et R' forment ensemble et avec les atomes de soufre qui les portent une molécule cyclique comportant de 4 à 22 atomes.

2. Procédé selon la revendication 1, comprenant au moins les étapes de :
a') préparation d'un mélange comprenant :
• un disulfure de formule R-S-S-R',
• une quantité catalytique d'acide aminé porteur d'un groupement thiol ou d'un peptide à groupement thiol,
• une quantité catalytique d'enzyme réductase correspondant audit acide aminé porteur d'un groupement thiol ou audit peptide à groupement thiol,
• une quantité catalytique de NADPH,
b') ajout d'un composé organique réducteur en quantité stœchiométrique par rapport au disulfure avec une quantité catalytique de l'enzyme déshydrogénase correspondante,
c') conduite de la réaction enzymatique,
d') récupération du mercaptan de formule R-SH et du mercaptan de formule R'-SH,
e') séparation et purification éventuelle du mercaptan de formule R-SH et du mercaptan de formule R'-SH.

3. Procédé selon l'une quelconque des revendication précédentes, dans lequel les radicaux R et R', identiques ou différents, sont choisis indépendamment l'un de l'autre, parmi les radicaux alkyle , alkylaryle, comportant de 1 à 20 atomes de carbone, , linéaires ou ramifiés, saturés ou non, et éventuellement fonctionnalisés par une ou plusieurs fonctions choisies parmi les fonction alcool, aldéhyde, cétone, acide, amide, nitrile, ester ou encore les fonctions porteuses de soufre, phosphore, silicium ou halogène.

4. Procédé selon l'une quelconque des revendications précédentes, dans lequel le disulfure de formule R-S-S-R' est le disulfure de diméthyle.

5. Procédé selon l'une quelconque des revendications précédentes, dans lequel l'acide aminé porteur de groupement thiol ou le peptide porteur de groupement thiol est choisi parmi la cystéine, l'homocystéine, le glutathion et la thiorédoxine.

6. Procédé selon l'une quelconque des revendications précédentes, dans lequel le composé organique réducteur est un composé organique réducteur donneur d'hydrogène porteur de fonction hydroxyle, choisi parmi les alcools, les polyols, les sucres.

7. Procédé selon l'une quelconque des revendications précédentes, dans lequel le composé organique réducteur est choisi parmi le glucose, le glucose-6-phosphate et l'isopropanol.

8. Procédé selon l'une quelconque des revendications précédentes, dans lequel le pH de la réaction est compris entre 6 et 8.

9. Procédé selon l'une quelconque des revendications précédentes, dans lequel le pH de la réaction est compris entre 6,5 et 7,5.

10. Procédé selon l'une quelconque des revendications précédentes, dans lequel le ratio molaire composé organique réducteur/disulfure est compris entre 0,01 et 100, globalement sur l'ensemble de la réaction.

11. Procédé selon l'une quelconque des revendications précédentes, dans lequel le ratio molaire composé organique réducteur/disulfure est compris entre 0,5 et 5 globalement sur l'ensemble de la réaction.

12. Procédé selon l'une quelconque des revendications précédentes, dans lequel le ratio molaire composé organique réducteur/disulfure est égal à 1 globalement sur l'ensemble de la réaction.

13. Mélange comprenant :
• du diméthyldisulfure,
• une quantité catalytique d'acide aminé porteur d'un groupement thiol ou d'un peptide à groupement thiol,
• une quantité catalytique d'une enzyme catalysant la réduction du pont disulfure créé entre deux équivalents dudit acide aminé porteur d'un groupement thiol ou audit peptide à groupement thiol,
• une quantité catalytique d'une enzyme catalysant la déshydrogénation d'un composé organique réducteur,
• une quantité catalytique d'un cofacteur commun aux deux enzymes catalysant la réduction et la déshydrogénation.

14. Mélange selon la revendication 13 comprenant :
• du diméthyldisulfure,
• une quantité catalytique d'acide aminé porteur d'un groupement thiol ou d'un peptide à groupement thiol,
• une quantité catalytique d'enzyme réductase correspondant audit acide aminé porteur d'un groupement thiol ou audit peptide à groupement thiol, et
• une quantité catalytique de NADPH, et
• une quantité catalytique d'une enzyme deshydrogénase.

## Patentansprüche

1. Verfahren zur Herstellung eines Mercaptans der Formel R-SH, wobei es zumindest die folgenden Schritte umfasst:
a) Herstellen einer Mischung, die Folgendes umfasst:
1) ein Disulfid der Formel R-S-S-R',
2) eine katalytische Menge an Aminosäure, die mit einer Thiolgruppe versehen ist, oder eines Peptids mit Thiolgruppe,
3) eine katalytische Menge eines Enzyms, das die Reduktion der Disulfidbrücke katalysiert, welche zwischen zwei Äquivalenten der Aminosäure, die mit einer Thiolgruppe versehen ist, oder des Peptids mit Thiolgruppe entstanden ist,
4) eine katalytische Menge eines Enzyms, welches die Dehydrierung der reduzierenden organischen Verbindung katalysiert, die am Schritt b) beteiligt ist,
5) eine katalytische Menge eines Cofaktors, der den beiden Enzymen gemein ist, welche die Reduktion und die Dehydrierung katalysieren,
b) Hinzufügen einer reduzierenden organischen Verbindung in stöchiometrischer Menge unter Bezugnahme auf das Disulfid der Formel R-S-S-R',
c) Durchführen der enzymatischen Reaktion,
d) Gewinnen des Mercaptans der Formel R-SH und des Mercaptans der Formel R'-SH,
e) möglicherweise Abtrennen und möglicherweise Aufreinigen des Mercaptans der Formel R-SH und/oder des Mercaptans der Formel R'-SH;
wobei R und R', die vollkommen gleichartig oder verschiedenartig sein können, für einen Kohlenwasserstoffrest geradkettiger oder verzweigter Art stehen, der 1 bis 20 Kohlenstoffatome aufweist, wobei die Kette gesättigt ist oder mit einer oder mehreren ungesättigten Stellen in Form von (einer) Doppeloder Dreifachbindung(en) versehen ist, oder R und R' zusammen mit den Schwefelatomen, an welche sie gebunden sind, ein zyklisches Molekül bilden, das 4 bis 22 Atome aufweist.

2. Verfahren nach Anspruch 1, das zumindest die folgenden Schritte umfasst:
a') Herstellen einer Mischung, die Folgendes umfasst:
• ein Disulfid der Formel R-S-S-R',
• eine katalytische Menge an Aminosäure, die mit einer Thiolgruppe versehen ist, oder eines Peptids mit Thiolgruppe,
• eine katalytische Menge an Reductase-Enzym, welches der Aminosäure, die mit einer Thiolgruppe versehen ist, oder dem Peptid mit Thiolgruppe entspricht,
• eine katalytische Menge an NADPH,
b') Zusetzen einer reduzierenden organischen Verbindung in stöchiometrischer Menge unter Bezugnahme auf das Disulfid, mit einer katalytischen Menge des entsprechenden Dehydrogenase-Enzyms,
c') Durchführen der enzymatischen Reaktion,
d') Gewinnen des Mercaptans der Formel R-SH und des Mercaptans der Formel R'-SH,
e') Abtrennen und möglicherweise Aufreinigen des Mercaptans der Formel R-SH und des Mercaptans der Formel R'-SH.

3. Verfahren nach einem beliebigen der vorhergehenden Ansprüche, wobei die Reste R und R', die vollkommen gleichartig oder verschiedenartig sind, unabhängig voneinander aus den Resten Alkyl, Alkylaryl ausgewählt sind, wobei diese 1 bis 20 Kohlenstoffatome aufweisen, geradkettig oder verzweigt sind, gesättigt sind oder nicht, und möglicherweise mit einer oder mehreren funktionellen Gruppen funktionalisiert sind, die aus den funktionellen Gruppen Alkohol, Aldehyd, Keton, Säure, Amid, Nitril, Ester oder auch den funktionellen Gruppen ausgewählt sind, welche mit Schwefel, Phosphor, Silicium oder Halogen versehen sind.

4. Verfahren nach einem beliebigen der vorhergehenden Ansprüche, wobei es sich bei dem Disulfid der Formel R-S-S-R' um Dimethyldisulfid handelt.

5. Verfahren nach einem beliebigen der vorhergehenden Ansprüche, wobei die Aminosäure, welche mit einer Thiolgruppe versehen ist, oder das Peptid, welches mit einer Thiolgruppe versehen sind, aus Cystein, Homocystein, Glutathion und Thioredoxin ausgewählt ist.

6. Verfahren nach einem beliebigen der vorhergehenden Ansprüche, wobei es sich bei der reduzierenden organischen Verbindung um eine wasserstoffspendende reduzierende organische Verbindung handelt, die mit einer funktionellen Hydroxylgruppe versehen ist, wobei sie aus den Alkoholen, den Polyolen, den Zuckern ausgewählt ist.

7. Verfahren nach einem beliebigen der vorhergehenden Ansprüche, wobei die reduzierende organische Verbindung aus Glucose, Glucose-6-Phosphat und Isopropanol ausgewählt ist.

8. Verfahren nach einem beliebigen der vorhergehenden Ansprüche, wobei der pH-Wert des Reaktionsansatzes im Bereich von 6 bis 8 liegt.

9. Verfahren nach einem beliebigen der vorhergehenden Ansprüche, wobei der pH-Wert des Reaktionsansatzes im Bereich von 6,5 bis 7,5 liegt.

10. Verfahren nach einem beliebigen der vorhergehenden Ansprüche, wobei das Molverhältnis reduzierende organische Verbindung / Disulfid im Bereich von 0,01 bis 100 liegt, bezogen auf die Gesamtheit des vollständigen Reaktionsansatzes.

11. Verfahren nach einem beliebigen der vorhergehenden Ansprüche, wobei das Molverhältnis reduzierende organische Verbindung / Disulfid im Bereich von 0,5 bis 5 liegt, bezogen auf die Gesamtheit des vollständigen Reaktionsansatzes.

12. Verfahren nach einem beliebigen der vorhergehenden Ansprüche, wobei das Molverhältnis reduzierende organische Verbindung / Disulfid gleich 1 ist, bezogen auf die Gesamtheit des vollständigen Reaktionsansatzes.

13. Mischung, umfassend:
• Dimethyldisulfid,
• eine katalytische Menge an Aminosäure, die mit einer Thiolgruppe versehen ist, oder eines Peptids mit Thiolgruppe,
• eine katalytische Menge eines Enzyms, das die Reduktion der Disulfidbrücke katalysiert, welche zwischen zwei Äquivalenten der Aminosäure, die mit einer Thiolgruppe versehen ist, oder am Peptid mit Thiolgruppe entstanden ist,
• eine katalytische Menge eines Enzyms, welches die Dehydrierung einer reduzierenden organischen Verbindung katalysiert,
• eine katalytische Menge eines Cofaktors, der den beiden Enzymen gemein ist, welche die Reduktion und die Dehydrierung katalysieren.

14. Mischung nach Anspruch 13, die Folgendes umfasst:
• Dimethyldisulfid,
• eine katalytische Menge an Aminosäure, die mit einer Thiolgruppe versehen ist, oder eines Peptids mit Thiolgruppe,
• eine katalytische Menge an Reductase-Enzym, welches der Aminosäure, die mit einer Thiolgruppe versehen ist, oder dem Peptid mit Thiolgruppe entspricht, und
• eine katalytische Menge an NADPH, und
• eine katalytische Menge eines Dehydrogenase-Enzyms.

## Claims

1. Process for the preparation of a mercaptan of formula R-SH, comprising at least the steps of:
a) preparation of a mixture comprising:
1) a disulfide of formula R-S-S-R',
2) a catalytic amount of an amino acid bearing a thiol group or of a thiol-group-containing peptide,
3) a catalytic amount of an enzyme catalysing the reduction of the disulfide bridge created between two equivalents of said amino acid bearing a thiol group or of said thiol-group-containing peptide,
4) a catalytic amount of an enzyme catalysing the dehydrogenation of the organic reducing compound involved in step b),
5) a catalytic amount of a cofactor common to the two enzymes catalysing the reduction and the dehydrogenation,
b) addition of an organic reducing compound in a stoichiometric amount relative to the disulfide of formula R-S-S-R',
c) carrying out the enzymatic reaction,
d) recovery of the mercaptan of formula R-SH and of the mercaptan of formula R'-SH,
e) optional separation and optional purification of the mercaptan of formula R-SH and/or of the mercaptan of formula R'-SH;
in which R and R', which are identical or different, represent independently of one another a linear or branched hydrocarbon-based radical comprising from 1 to 20 carbon atoms, said chain being saturated or bearing one or more unsaturations in the form of double or triple bond(s), or R and R' form together, and with the sulfur atoms bearing them, a cyclic molecule comprising from 4 to 22 atoms.

2. Process according to Claim 1, comprising at least the steps of:
a') preparation of a mixture comprising:
• a disulfide of formula R-S-S-R',
• a catalytic amount of amino acid bearing a thiol group or of a thiol-group-containing peptide,
• a catalytic amount of reductase enzyme corresponding to said amino acid bearing a thiol group or to said thiol-group-containing peptide,
• a catalytic amount of NADPH,
b') addition of an organic reducing compound in a stoichiometric amount relative to the disulfide with a catalytic amount of the corresponding dehydrogenase enzyme,
c') carrying out the enzymatic reaction,
d') recovery of the mercaptan of formula R-SH and of the mercaptan of formula R'-SH,
e') separation and optional purification of the mercaptan of formula R-SH and of the mercaptan of formula R'-SH.

3. Process according to either one of the preceding claims, in which the radicals R and R', which are identical or different, are chosen, independently of one another, from linear or branched, saturated or unsaturated alkyl or alkylaryl radicals comprising from 1 to 20 carbon atoms, and optionally functionalized with one or more functions chosen from alcohol, aldehyde, ketone, acid, amide, nitrile, or ester functions or else functions bearing sulfur, phosphorus, silicon or a halogen.

4. Process according to any one of the preceding claims, in which the disulfide of formula R-S-S-R' is dimethyl disulfide.

5. Process according to any one of the preceding claims, in which the amino acid bearing a thiol group or the peptide bearing a thiol group is chosen from cysteine, homocysteine, glutathione and thioredoxin.

6. Process according to any one of the preceding claims, in which the organic reducing compound is a hydrogen-donating organic reducing compound bearing a hydroxyl function, chosen from alcohols, polyols and sugars.

7. Process according to any one of the preceding claims, wherein the organic reducing compound is chosen from glucose, glucose-6-phosphate and isopropanol.

8. Process according to any one of the preceding claims, in which the pH of the reaction is between 6 and 8.

9. Process according to any one of the preceding claims, in which the pH of the reaction is between 6.5 and 7.5.

10. Process according to any one of the preceding claims, in which the organic reducing compound/disulfide molar ratio is between 0.01 and 100, overall over the whole of the reaction.

11. Process according to any one of the preceding claims, in which the organic reducing compound/disulfide molar ratio is between 0.5 and 5 overall over the whole of the reaction.

12. Process according to any one of the preceding claims, in which the organic reducing compound/disulfide molar ratio is equal to 1 overall over the whole of the reaction.

13. Mixture comprising:
• dimethyl disulfide,
• a catalytic amount of an amino acid bearing a thiol group or of a thiol-group-containing peptide,
• a catalytic amount of an enzyme catalysing the reduction of the disulfide bridge created between two equivalents of said amino acid bearing a thiol group or to said thiol-group-containing peptide,
• a catalytic amount of an enzyme catalysing the dehydrogenation of an organic reducing compound,
• a catalytic amount of a cofactor common to the two enzymes catalysing the reduction and the dehydrogenation.

14. Mixture according to Claim 13, comprising:
• dimethyl disulfide,
• a catalytic amount of amino acid bearing a thiol group or of a thiol-group-containing peptide,
• a catalytic amount of reductase enzyme corresponding to said amino acid bearing a thiol group or to said thiol-group-containing peptide, and
• a catalytic amount of NADPH, and
• a catalytic amount of a dehydrogenase enzyme.
